(19) Europäisches Patentamt
European Patent Office
Office européen des brevets

(11) Numéro de publication: **0 118 354**
**B1**

(12) **FASCICULE DE BREVET EUROPEEN**

(45) Date de publication du fascicule du brevet: **23.10.85**

(51) Int. Cl.[4]: **C 07 C 13/277**, C 07 C 11/21, C 07 C 7/148

(21) Numéro de dépôt: **84400365.7**

(22) Date de dépôt: **23.02.84**

(54) **Procédé chimique de purification d'un mélange de trimères de l'isoprène.**

(30) Priorité: **24.02.83 FR 8303002**

(43) Date de publication de la demande: **12.09.84 Bulletin 84/37**

(45) Mention de la délivrance du brevet: **23.10.85 Bulletin 85/43**

(84) Etats contractants désignés: **AT BE CH DE FR GB IT LI LU NL SE**

(56) Documents cités: **EP - A - 0 044 771**

(73) Titulaire: **RHONE-POULENC SANTE, Les Miroirs 18 Avenue d'Alsace, F-92400 Courbevoie Cedex (FR)**

(72) Inventeur: **Morel, Didier, 19 avenue Jean Mermoz, F-69008 Lyon (FR)**

(74) Mandataire: **Pilard, Jacques et al, RHONE-POULENC RECHERCHES Service Brevets Pharma 25, Quai Paul Doumer, F-92408 Courbevoie Cedex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 118 354 B1

BUNDESDRUCKEREI BERLIN

**Description**

La présente invention concerne un procédé de purification par voie chimique d'un mélange de trimères de l'isoprène.

La trimérisation de l'isoprène, en présence d'un catalyseur constitué d'un sel de nickel tel que l'acétylacétonate de nickel, d'une amine telle que la pyridine et d'un réducteur tel que le triéthylaluminium à 80°C, fournit approximativement 15% de dimères de l'isoprène (coupe $C_{10}$), 65% de trimères (coupe $C_{15}$) et 20% de produits lourds.

La coupe $C_{15}$, qui peut être facilement séparée par distillation, est constituée essentiellement de trimères cycliques (triméthylcyclododécatriènes) et de trimères linéaires caractérisés par les structures

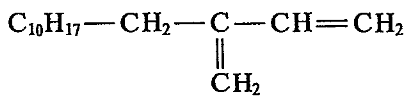 (I)

et

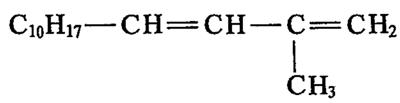 (II)

Parmi les trimères linéaires correspondant à la structure (I), le $\beta$-farnésène est particulièrement intéressant comme intermédiaire de synthèse de la viatmine E en passant par la farnésylacétone et la phytone.

Il est connu, d'après S. Akutagawa et coll., Bull. Soc. Chim. Japan, 51, 1158–1162 (1978) de transformer le $\beta$-farnésène en farnésylacétone par addition d'acide chlorhydrique en présence de chlorure de cuivre suivie d'une condensation sur l'acétylacétate de méthyle en présence d'une base forte puis décarboxylation. Cependant l'addition d'acide chlorhydrique sur les diènes-1,3 n'est pas sélective, l'addition s'effectuant indifféremment sur les enchaînements

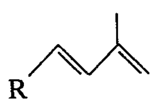 ou 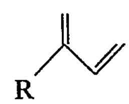

Il en résulte que la mise en oeuvre du procédé nécessite l'utilisation de $\beta$-farnésène pur en vue d'obtenir la farnésylacétone ayant la pureté désirée.

Il est connu également, d'après la demande de brevet européen publiée sous le numéro 44 771 de préparer la farnésylacétone par action de l'acétylacétate de méthyle sur le $\beta$-farnésène en présence d'une phosphine soluble dans l'eau et d'un catalyseur choisi parmi les sels inorganiques et organiques et les complexes du rhodium puis décarboxylation. L'addition sélective de l'acétylacétate de méthyle nécessite cependant l'utilisation de $\beta$-farnésène purifié.

Ces procédés connus de préparation de la farnésylacétone à partir du $\beta$-farnésène impliquent donc la séparation du $\beta$-farnésène de la coupe $C_{15}$ des trimères de l'isoprène. Cependant la distillation fractionnée de la coupe $C_{15}$ des trimères de l'isoprène, qui contient approximativement 45% de $\beta$-farnésène, ne permet pas d'obtenir le $\beta$-farnésène avec des rendements suffisants. En effet, les points d'ébullition des différents trimères sont très voisins et il se forme des polymères par chauffage prolongé au cours de la distillation.

Il a maintenant été trouvé, et c'est ce qui constitue l'objet de la présente invention que, parmi les isomères linéaires constituant la coupe $C_{15}$, seuls ceux possédant l'enchaînement terminal

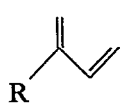

réagissent, dans les conditions décrites dans la demande de brevet européen publiée sous le numéro 44 771, avec un composé ayant un atome de carbone activé de formule générale:

$$X-CH_2-Y \qquad (III)$$

dans laquelle l'un des symboles X ou Y est un groupement électroattracteur et l'autre représente un groupement électroattracteur ou électrodonneur. Il en résulte que le composé de formule générale (III) réagit avec les trimères de structure (I) donnée précédemment contenus dans la coupe $C_{15}$, séparée par distillation de la coupe $C_{10}$ et des produits lourds, sans toucher aux trimères de structure (II) ou aux trimères cycliques.

Selon l'invention, le traitement de la coupe $C_{15}$ par un produit de formule générale (III) permet d'obtenir les produits de structure:

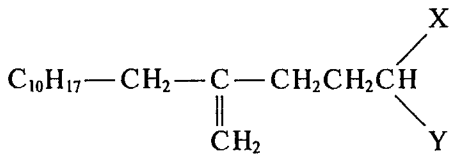

(IV)

et

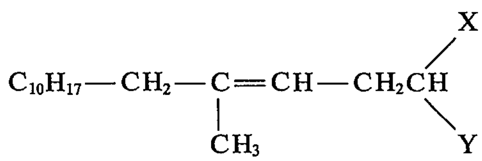

(V)

dans lesquelles X et Y sont définis comme précédemment, qui sont alors facilement séparés des trimères cycliques et des trimères linéaires de structure (II) donnée précédemment.

Pour la mise en oeuvre du procédé selon l'invention, la coupe $C_{15}$ est traitée par un produit de formule générale (III) en présence d'un catalyseur constitué, d'une part, d'une phosphine soluble dans l'eau et, d'autre part, par au moins un composé du rhodium choisi parmi les sels inorganiques, organiques et les complexes du rhodium en opérant dans l'eau ou dans un mélange hydro-alcoolique contenant au maximum 50% d'un alcool aliphatique contenant 1 à 3 atomes de carbone, puis le mélange réactionnel est fractionné, par exemple, par distillation de façon à séparer les produits de formules (IV) et (V) des trimères cycliques et des trimères linéaires de structure (II).

Dans le produit de formule (III) un des symboles X et Y est choisi parmi le groupe électroacttracteur comprenant les radicaux de formule CHO, $COR_1$, $CO_2R_2$, $SO_2R_3$, $CONR_4R_5$, CN, $NO_2$ et l'autre est choisi parmi le groupe comprenant les radicaux de formules $NHCOR_7$, $OR_8$, OH, $OCOR_9$, $SR_{10}$, SH, les atomes d'halogène dans lesquels $R_1$, $R_2$, $R_3$, $R_4$, $R_5$, $R_7$, $R_8$, $R_9$ et $R_{10}$ représentent un radical hydrocarboné contenant 1 à 12 atomes de carbone. Lorsque X et Y représentent l'un un groupement électroattracteur et l'autre un groupement électrodonneur, ils doivent être choisis de telle manière que l'effet du groupement électroattracteur soit prépondérant.

On peut citer comme exemples de composés III les composés suivants: la pentanedione-2,4 $CH_3COCH_2COCH_3$; la butanal-1 one-3 $CH_3COCH_2CHO$; l'acétyl acétate d'éthyle $CH_3COCH_2COOC_2H_5$; l'acétyl acétate de méthyle $CH_3COCH_2COOCH_3$, le phénylsulfonyl acétone $C_6H_5SO_2CH_2COCH_3$, la phénylsulfonylacétate d'éthyle $C_6H_5SO_2CH_2CO_2C_2H_5$, l'éthylsulfonylacétone $C_2H_5SO_2CH_2COCH_3$, $CH_3COCH_2CON(CH_3)_2$, $CH_3COCH_2CON(CH_3)C_2H_5$, le cyanoacétate d'éthyle $NCCH_2CO_2C_2H_5$, la cyanoacétone $NCCH_2COCH_3$, le nitroacétate d'éthyle $NO_2CH_2CO_2C_2H_5$ la nitroacétone $NO_2CH_2COCH_3$, le malonate de diéthyle $CH_2(CO_2C_2H_5)_2$, l'hydroxyacétone $HOCH_2COCH_3$.

Les phosphines solubles dans l'eau utilisables dans le cadre de la présente invention sont celles décrites dans le brevet français 7 622 824 (2 366 237).

Plus particulièrement, on préfère utiliser au moins une phosphine de formule:

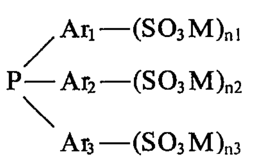

(VI)

dans laquelle:

- $Ar_1$, $Ar_2$ et $Ar_3$ identiques ou différents représentent chacun un radical choisi parmi le groupe comprenant les radicaux phénylène et les radicaux naphthylène, ces radicaux étant éventuellement substitués.
- M est un reste cationique d'origine minérale ou organique choisi de manière que la phosphine de formule (VI) soit soluble dans l'eau.
- $n_1$, $n_2$ et $n_3$ identiques ou différents sont des nombres entiers supérieurs ou égaux à 0 et inférieurs ou égaux à 3, l'un au moins étant supérieur ou égal à 1.

Les radicaux phénylène et naphthylène peuvent être substitués par tous radicaux ne contrariant pas la solubilité de la phosphine (VI) dans l'eau. Parmi ceux-ci, on peut citer, à titre d'exemples, les radicaux alkyle ayant de 1 à 6 atomes de carbone, les radicaux alkoxy ayant de 1 à 6 atomes de carbone, les atomes d'halogène, les radicaux $-OH$, $-CN$, $-NO_2$, $-N-(alkyl)_2$, carboxylates.

Le procédé selon l'invention est de préférence mis en oeuvre en utilisant au moins une phosphine de formule (VI) dans laquelle $Ar_1$, $Ar_2$ et $Ar_3$ identiques ou différents représentent chacun un radical choisi

parmi le groupe comprenant le radical phénylène et les radicaux phénylène substitués.

Encore plus préférentiellement, on utilise une phosphine dans laquelle au moins un des groupements $SO_3M$ est en position méta sur le noyau benzénique.

De préférence, M est choisi parmi le groupe comprenant les cations dérivés des métaux Na, K, Ca, Ba, les ions $NH_4^+$ et ammonium quaternaire comme les ions tétraméthylammonium, tétrapropylammonium et tétrabutylammonium.

$n_1$, $n_2$ et $n_3$ sont de préférence égaux à 0 ou 1, $n_1 + n_2 + n_3$ étant compris entre 1 et 3 ($1 \leq n_1 + n_2 + n_3 \leq 3$).

Les composés de formule VI plus particulièrement préférés sont les phosphines de formules suivantes:

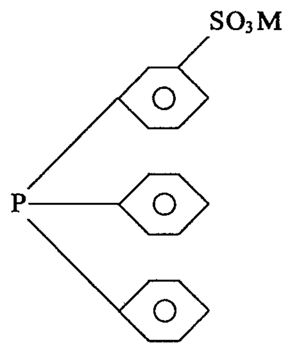

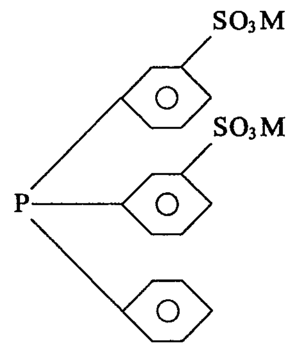

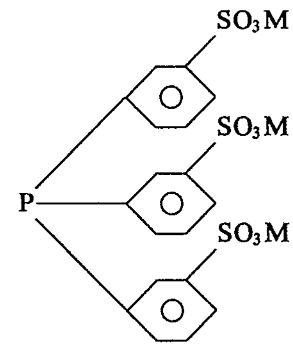

où M a la signification précédente.

On peut citer comme autres exemples de phosphine (VI) pouvant être utilisées dans le procédé selon l'invention les composés suivants: les sels alcalins ou alcalino-terreux, les sels d'ammonium, les sels d'ammonium quaternaires des (p-sulfophényl) diphénylphosphine; (m-sulfo p-méthylphényl)di(p-méthylphényl) phosphine; (m-sulfo p-méthoxyphényl)di(p-méthoxyphényl)phosphine; (m-sulfo p- chlorophényl)di(p-chlorophényl) phosphine; di(p-sulfophényl) phénylphosphine; di(m-sulfo p-méthylphényl) (p-méthylphényl) phosphine; di(m-sulfo p-méthoxyméthyl) (p-méthoxyphényl) phosphine; di (m-sulfo p-chlorphényl) (p-chlorophényl) phosphine; tri(p-sulfophényl) phosphine; tri(m-sulfo p-méthylphényl) phosphine; tri(m-sulfo p-méthoxyphényl) phosphine; tri(m-sulfo p-méthoxyphényl) phosphine; tri(m-sulfo p-chlorophényl) phosphine; (o-sulfo p-méthylphényl) (m-sulfo p-méthyl) (m, m'-disulfo p-méthyl) phosphine; (m-sulfophényl) (m-sulfo p-chlorophényl) (m, m'-disulfo p-chlorophényl) phosphine.

Le composé du rhodium utilisé doit être soluble dans l'eau ou capable de passer en solution dans l'eau dans les conditions de la réaction, par réaction de coordination avec les phosphines hydrosolubles. Généralement le composé du rhodium est choisi parmi le groupe comprenant par exemple $RhCl_3$, $RhBr_3$, $Rh_2O$, $Rh_2O_3$, $Rh(NO_3)_3$, $Rh(CH_3COO)_3$, $Rh(CH_3COCHCOCH_3)_3$, $[RhCl(cyclooctadiène\text{-}1,5)]_2$, $[RhCl(CO)_2]_2$ ou $RhCl_3(C_2H_5NH_2)_3$.

On utilise une quantité de rhodium telle que le nombre d'atomes-gramme de rhodium élémentaire par litre de solution réactionnelle soit compris entre $10^{-4}$ et 1.

La quantité de phosphine est choisie de telle sorte que le nombre d'atomes-gramme de phosphore trivalent rapporté à un atome-gramme de rhodium soit compris entre 0,1 et 200.

Bien que cela ne soit pas impératif, on peut ajouter au mélange réactionnel un réducteur du rhodium tel que le borohydrure de sodium, la poudre de zinc, le borohydrure de potassium, le magnésium et l'hydrazine.

Selon un mode de mise en oeuvre particulier mais non obligatoire de l'invention, on ajoute au milieu réactionnel une base dans le but d'améliorer la réactivité. On peut citer comme exemples de bases convenant particulièrement bien, les hydroxydes, carbonates et bicarbonates des métaux alcalins et alcalino-terreux et les amines tertiaires aliphatiques ou aromatiques. On utilise de préférence entre 0,005 et 5 moles de base/litre de solution aqueuse.

La température à laquelle est conduite la réaction peut varier dans de larges limites. Préférentiellement, on opère à des températures modérées inférieures à 200°C. Plus particulièrement, la température est comprise entre 50°C et 125°C.

La quantité d'eau minimum nécessaire est celle suffisante pour dissoudre le catalyseur en totalité et au moins une partie des réactifs, la réaction ayant lieu en phase aqueuse, les produits de la réaction étant en phase organique non miscible à l'eau.

Afin d'augementer la cinétique de la réaction et de faciliter le recyclage du catalyseur, il est possible d'opérer en présence d'un co-solvant. Plus particulièrement une partie de l'eau nécessaire à la mise en oeuvre de la réaction est remplacée par une quantité équivalente d'un alcool aliphatique contenant 1 à 3 atomes de carbone, tel que le méthanol, l'éthanol ou l'isopropanol. La quantité d'eau qui peut être remplacée est au maximum égale à la la moitié de la quantité d'eau nécessaire à la mise en oeuvre de la réaction sans co-solvant.

Une manière pratique de mettre en oeuvre le procédé de l'invention consiste à charger dans un réacteur convenable après l'avoir purgé a l'aide d'un gaz inerte (azote, argon) soit la solution aqueuse ou hydroalcoolique catalytique préalablement formée, soit les divers composants: phosphine, eau, éventuellement l'alcool tel que le méthanol, l'éthanol ou l'isopropanol, composé du rhodium avec éventuellement le réducteur et la base. On porte le réacteur à la température de réaction avant ou après l'introduction du composé ayant un atome de carbone activé qui lui-même peut être introduit avant, après ou simultanément à la coupe $C_{15}$ à traiter.

Après arrêt de la réaction, on refroidit à la température ambiante. Le contenu du réacteur est soutiré et on isole ensuite le produit de la réaction qui est en phase organique en séparant cette dernière de la phase aqueuse contenant le catalyseur par décantation et éventuellement par une extraction à l'aide d'un solvant convenable.

La solution aqueuse ou hydroalcoolique résiduelle peut être recyclée dans le réacteur pour catalyser une nouvelle réaction. En particulier, en solution hydro-méthanolique le catalyseur peut être recyclé plus de 25 fois sans perte d'activité. La solution aqueuse ou hydroalcoolique peut aussi rester dans le réacteur, les produits organiques étant alors soutirés après décantation.

Le procédé selon la présente invention permet d'obtenir les produits de structure (IV) et (V) avec un taux de transformation du trimère de structure (I) généralement supérieur à 90% et une sélectivité généralement supérieure à 95%.

Lorsque l'on utilise, comme composé à groupement méthylène activé de formule générale (III), l'acétylacétate de méthyle, les produits obtenus peuvent être décarboxylés puis séparés des trimères n'ayant pas réagi par exemple par distillation. Les cétones éthyléniques ainsi obtenues peuvent ensuite être hydrogénées, par exemple en présence de palladium sur charbon, pour donner les cétones saturées correspondantes dont la phytone qui est utilisée dans la synthèse de la vitamine E.

Les exemples suivants, donnés à titre non limitatif, montrent comment l'invention peut être mise en pratique.

## Exemple 1

Dans un autoclave en acier inoxydable préalablement purgé à l'argon, on additionne successivement 0,0406 g de $[RhCl(cyclooctadiène\text{-}1{,}5)]_2$ (0,105 milliatome-gramme de rhodium), 0,6322 g de

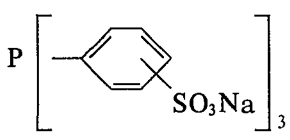

(que l'on appellera dans les exemples suivants TPPTS Na), soit 0,83 milliatome-gramme de $P^{+3}$, 0,101 g de $Na_2CO_3$ (0,95 millimole), 15 cm3 d'eau et 5 cm3 de méthanol. Puis on ajoute 10,76 g d'acétylacétate de méthyle (92,8 millimoles) et 6,74 g de coupe $C_{15}$ contenant 2,58 g de réactifs de formule I (12,65 millimoles), 2,7 g d'oléfines de formule II (13,2 millimoles) et 1,46 g de triméthylcyclododécatriènes.

On chauffe à 80°C sous agitation pendant 16 heures.

Après séparation, par décantation, de la phase aqueuse contenant le catalyseur, on récupère 7,98 g de phase organique exempte d'acétylacétate de méthyle et contenant

- 0,26 g de réactif de formule I
- 2,7 g d'oléfines de formule II
- 1,46 g de triméthylcyclododécatriènes
- 3,57 g d'un mélange équimolaire des produits a et a'

de formule:

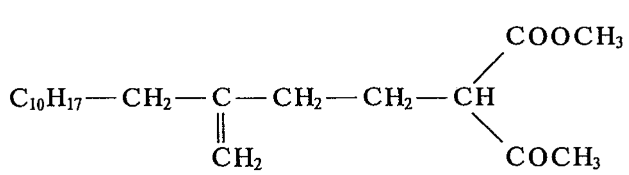

(a)

et

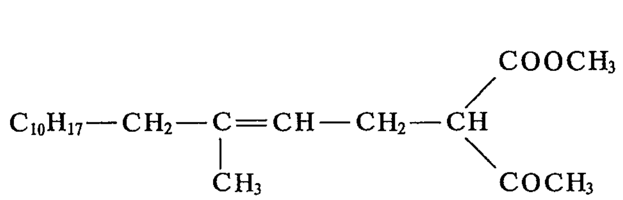

(a')

ce qui correspond à un taux de transformation de I de 90%, de l'acétylacétate de méthyle de 12%; la sélectivité en (a) + (a') étant de 98%.

Après traitement du mélange des produits a + a' par la soude aqueuse puis par l'acide sulfurique, on obtient 2,83 g d'un mélange de cétones c + c' répondant aux formules:

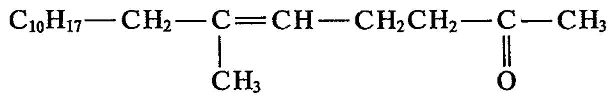

(c')

et

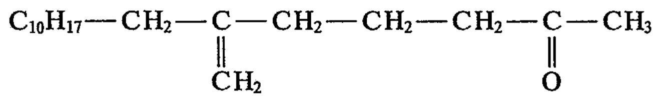

(c)

et 4,42 g d'oléfines.

La coupe $C_{15}$ peut être préparée de la manière suivante:

Dans un ballon de 100 cm3 préalablement purgé à l'argon on introduit 1,28 g d'acétylacétonate de nickel (5 mmoles) en solution à 4% dans le toluène, 15,7 g de pyridine (0,2 mole) et 10 cm3 d'isoprène (0,1 mole). Après refroidissement à −10°C, on ajoute lentement 15 mmoles de triéthylaluminium ($AlEt_3$) en solution dans le toluène. La solution, initialement de couleur bleu turquoise devient marron puis rouge après un réchauffement à 20°C.

Cette solution catalytique est introduite dans un réacteur cylindrique en acier inoxydable de 125 cm3 contenant 90 cm3 d'isoprène (0,9 mole). On chauffe sous agitation à 80°C pendant 8 heures.

Après refroidissement, le mélange réactionnel est versé dans 100 cm3 d'acide chlorhydrique 3 N puis on ajoute 100 cm3 de pentane. Après agitation jusqu'à décoloration et dissolution de la pyridine dans l'eau, le mélange réactionnel est décanté. La couche organique est concentrée. On obtient ainsi 21,5 g de couche organique contenant 5,24 g de toluène, 2,45 g de coupe $C_{10}$, 10,61 g de coupe $C_{15}$ dont 4,77 g de produit de structure I et 3,20 g de produits lourds (d'après l'analyse par chromatographie en phase gazeuse).

Par distillation, on obtient 6,74 g coupe $C_{15}$ contenant 2,58 g de produit de structure I.

## Exemple 2

Dans un autoclave en acier inoxydable préalablement purgé à l'argon, on introduit successivement:

- 0,0408 g de $[RhCl(cyclooctadiène\text{-}1,5)]_2$ (0,165 milliatome-gramme de Rh)
- 0,613 g de TPPTS Na (0,80 milliatome-gramme de $P^{+3}$)
- 0,2 g de $Na_2CO_3$ (1,89 millimole), 15 cm3 d'eau et 5 cm3 de méthanol.

Puis on ajoute 11,08 g d'acétylacétate de méthyle (95,5 millimoles) et 14,46 g de coupe $C_{15}$ contenant 4,9 g de réactifs de formule I (24 millimoles), 6,66 g d'oléfines de formule II (32,6 millimoles) et 2,9 g de triméthylcyclododécatriènes.

On chauffe à 80°C sous agitation pendant 14 heures.

Après séparation, par décantation, de la phase aqueuse contenant le catalyseur, on récupère 16,6 g de phase organique contenant:

- 0,78 g de réactif de formule I
- 6,66 g d'oléfines de formule II
- 2,9 g de triméthylcyclododécatriènes
- 6,26 g d'un mélange équimolaire des produits a et a'

ce qui correspond à un taux de transformation de I de 84%, de l'acétylacétate de méthyle de 20,5%; la sélectivité en (a) + (a') étant de 75%.

## Exemple 3

Dans un réacteur en acier inoxydable à agitation centrale préalablement purgé à l'argon, on introduit successivement:

- 0,0504 g de $[RhCl(cyclooctadiène\text{-}1,5)]_2$ (0,206 milliatome-gramme de Rh)
- 0,6 g de TPPTS Na (0,96 milliatome-gramme de $P^{+3}$)
- 0,101 g de $Na_2CO_3$ (0,95 millimole), 15 cm3 d'eau et 5 cm3 de méthanol.

Puis on ajoute 16,29 g d'acétylacétate de méthyle (140,43 millimoles) et 11,84 g de coupe $C_{15}$ contenant 1,47 g d'oléfines $C_{10}H_{16}$ (dimères de l'isoprène), 6,45 g de réactif de formule I (31,6 millimoles) et 4,14 g d'oléfines de formule II et de triméthylcyclododécatriènes.

On chauffe à 80°C sous agitation pendant 16 heures.

Après séparation, par décantation, de la phase aqueuse contenant le catalyseur, on récupère 14,62 g de phase organique concentrée contenant:

- 1,07 g d'oléfines $C_{10}H_{16}$
- 1,25 g de réactif de formule I
- 4,14 g d'oléfines de formule II + triméthylcyclododécatriènes
- 8,16 g d'un mélange équimolaire des produits (a) et (a') ce qui correspond à un taux de transformation de I de 80,7%, de l'acétylacétate de méthyle de 18,1%; la sélectivité en a + a' étant de 95%.

La phase organique est alors traitée pendant 20 heures à 20°C par un mélange contenant 30 cm3 de méthanol, 10 cm3 d'eau et 2 g d'hydroxyde de sodium (50 millimoles).

Puis, après élimination du méthanol sous pression réduite (100 mm de mercure; 13 kPa) on ajoute lentement 60 grammes d'une solution aqueuse d'acide sulfurique à 5% (30,6 millimoles).

Après décarboxylation complète, on récupère 13,14 g de phase organique contenant 6,68 g d'un mélange équimoléculaire des cétones (c) et (c').

Cette phase organique est alors mélangée à 40 cm3 de pentane et les doubles liaisons oléfiniques sont alors saturées par hydrogénation à 20°C sous une pression de 30 bar d'hydrogène en présence de 1 g de catalyseur constitué de palladium à 10% sur charbon.

On obtient alors 6,83 g d'un mélange de cétones correspondant à la formule:

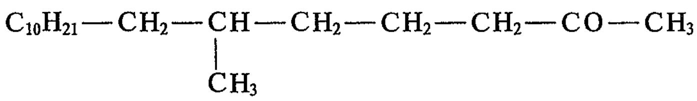

dont le spectre de RMN du $^{13}C$ montre la présence de 50% de phytone de formule:

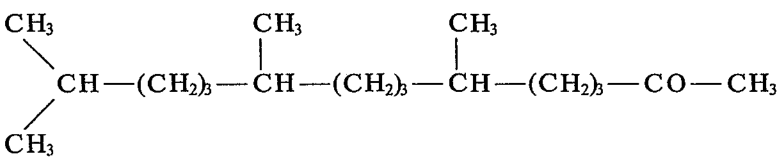

## Revendications

1. Procédé de purification par voie chimique d'un mélange de trimères de l'isoprène contenant essentiellement des trimères cycliques et des trimères linéaires caractérisés par les structures

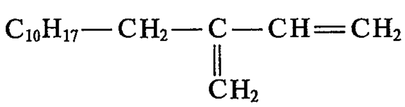

et

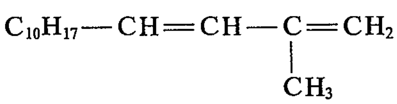

caractérisé en ce que l'on traite le mélange des trimères par un composé ayant un atome de carbone activé de formule générale:

$$X—CH_2—Y$$

dans laquelle l'un des symboles X ou Y est un groupement électroattracteur et l'autre représente un groupement électroattracteur ou électrodonneur en présence d'un catalyseur constitué, d'une part, d'une phosphine soluble dans l'eau et, d'autre part, d'un composé du rhodium dans l'eau ou dans un mélange hydroalcoolique contenant au maximum 50% d'un alcool aliphatique contenant 1 à 3 atomes de carbone, puis sépare par distillation les trimères cycliques et les trimères linéaires de structure

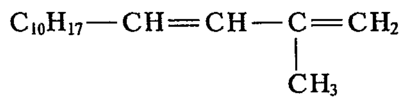

et récupère le mélange des produits de formule générale:

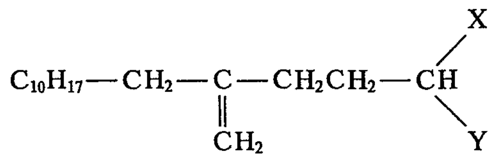

et

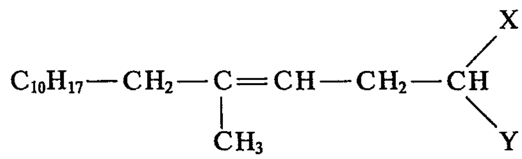

dans laquelle X et Y sont définis comme précédemment.

2. Procédé selon la revendication 1 caractérisé en ce que dans le composé ayant un atome de carbone activé de formule générale:

$$X-CH_2-Y$$

l'un des symboles X et Y est choisi parmi le groupe électroattracteur comprenant les radicaux de formule CHO, $COR_1$, $CO_2R_2$, $SO_2R_3$, $CONR_4R_5$, CN, $NO_2$ et l'autre est choisi parmi le groupe électroattracteur défini précédemment ou le groupe électrodonneur comprenant les radicaux de formule $NHCOR_7$, $OR_8$, OH, $OCOR_9$, $SR_{10}$, SH et les atomes d'halogène dans lesquels $R_1$, $R_2$, $R_3$, $R_4$, $R_5$, $R_7$, $R_8$, $R_9$ et $R_{10}$ représentent un radical hydrocarboné contenant 1 à 12 atomes de carbone.

3. Procédé selon l'une des revendication 1 ou 2 caractérisé en ce que le composé ayant un atome de carbone activé est choisi parmi la pentanedione-2,4, la butanal-1 one-3, l'acétylacétate d'éthyle, l'acétylacétate de méthyle, la phénylsulfonylacétone, le phénylsulfonylacétate d'éthyle, l'éthylsulfonylacétone, le cyano acétate d'éthyle, la cyanoacétone, le nitroacétate d'éthyle, la nitroacétone, le malonate de diéthyle ou l'hydroxyacétone.

4. Procédé selon l'une des revendication 1, 2 ou 3 caractérisé en ce que le composé ayant un atome de carbone activé est choisi parmi l'acétylacétate de méthyle ou l'acétylacétate d'éthyle.

**Patentansprüche**

1. Verfahren zum Reinigen einer Mischung von Isoprentrimeren, die im wesentlichen cyclische Trimere und lineare Trimere, gekennzeichnet durch die Formeln

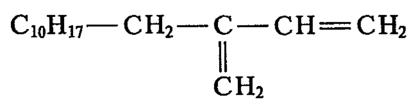

und

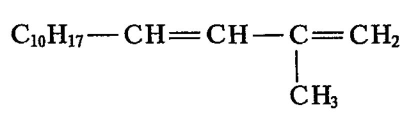

enthält, auf chemischem Wege, dadurch gekennzeichnet, daß man die Trimerenmischung mit einer ein aktiviertes Kohlenstoffatom enthaltenden Verbindung der allgemeinen Formel:

$$X-CH_2-Y$$

in welcher eines der Symbole X oder Y eine elektronenanziehende Gruppe ist und das andere eine elektronenanziehende oder elektronenabgegebende Gruppe darstellt, in Gegenwart eines Katalysators, bestehend aus einem wasserlöslichen Phosphin und anderseits aus einer Rhodiumverbindung in Wasser oder in einer wässerig-alkoholischen Mischung, die höchstens 50% eines aliphatischen Alkohols mit 1 bis 3 Kohlenstoffatomen enthält, behandelt und dann die cyclischen Trimeren und die linearen Trimeren der Formel

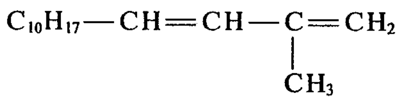

abdestilliert und die Mischung der Verbindungen der allgemeinen Formel:

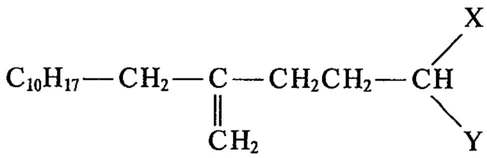

und

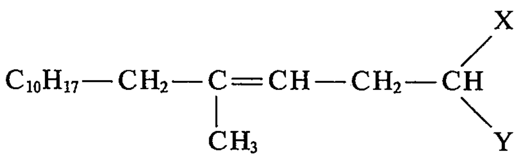

in welcher X und Y die obige Bedeutung haben, sammelt.

2. Verfahren nach Anspruch 1, dadurch gekennzeichnet, daß in der ein aktiviertes Kohlenstoffatom enthaltenden Verbindung der allgemeinen Formel:

$$X—CH_2—Y$$

eines der Symbole X und Y ausgewählt ist aus der elektronenanziehenden Gruppe, umfassend die Reste der Formel CHO, $COR_1$, $CO_2R_2$, $SO_2R_3$, $CONR_4R_5$, CN, $NO_2$, und das andere ausgewählt ist aus der oben definierten elektronenanziehenden Gruppe oder der elektronenabgebenden Gruppe, umfassend die Reste der Formel $NHCOR_7$, $OR_8$, OH, $OCOR_9$, $SR_{10}$, SH und die Halogenatome, worin $R_1$, $R_2$, $R_3$, $R_4$, $R_5$, $R_7$, $R_8$, $R_9$ und $R_{10}$ einen Kohlenwasserstoffrest mit 1 bis 12 Kohlenstoffatomen darstellen.

3. Verfahren nach einem der Ansprüche 1 oder 2, dadurch gekennzeichnet, daß die ein aktiviertes Kohlenstoffatom enthaltende Verbindung ausgewählt ist aus 2,4-Pentandion, 1-Butanal-3-on, Äthylacetylacetat, Methylacetylacetat, Phenylsulfonylaceton, Äthylphenylsulfonylacetat, Äthylsulfonylaceton, Äthylcyanoacetat, Cyanoaceton, Äthylnitroacetat, Nitroaceton, Diäthylmalonat oder Hydroxyaceton.

4. Verfahren nach einem der Ansprüche 1, 2 oder 3, dadurch gekennzeichnet, daß die ein aktiviertes Kohlenstoffatom enthaltende Verbindung ausgewählt ist aus Methylacetylacetat oder Äthylacetylacetat.

## Claims

1. Process for the chemical purification of a mixture of isoprene trimers essentially containing cyclic trimers and linear trimers characterised by the structures

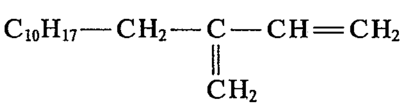

and

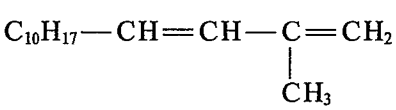

characterised in that the mixture of trimers is treated with a compound containing an activated carbon atom, of the general formula:

$$X—CH_2—Y$$

in which one of the symbols X or Y is an electron-attracting group and the other represents an electron-attracting or electron-donating group, in the presence of a catalyst consisting firstly of a water-soluble phosphine and secondly of a rhodium compound in water or in an aqueous-alcoholic mixture containing at most 50% of an aliphatic alcohol containing 1 to 3 carbon atoms, and in that thereafter the cyclic trimers of the structure

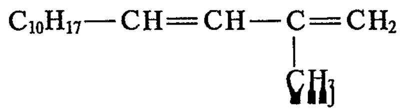

are separated off by distillation and the mixture of the products of the general formula:

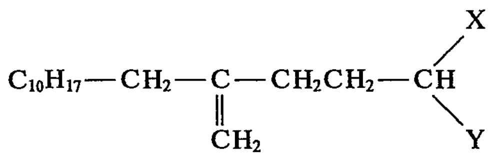

et

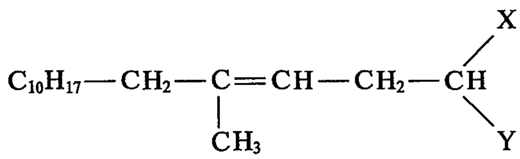

in which X and Y are defined as above, is recovered.

2. Process according to Claim 1, characterised in that in the compound having an activated carbon atom, of the gerneral formula:

$X—CH_2—Y$

one of the symbols X and Y is chosen from among the electronattracting group comprising the radicals of formula CHO, $COR_1$, $CO_2R_2$, $SO_2R_3$, $CONR_4R_5$, CN and $NO_2$ and the other is chosen from among the electron-attracting group defined above or from the electron-donating group comprising radicals of the formula $NHCOR_7$, $OR_8$, OH, $OCOR_9$, $SR_{10}$, SH and halogen atoms, in which $R_1$, $R_2$, $R_3$, $R_4$, $R_5$, $R_7$, $R_8$, $R_9$ and $R_{10}$ represent a hydrocarbon radical containing 1 to 12 carbon atoms.

3. Process according to one of Claims 1 or 2, characterised in that the compound having an activated carbon atom is chosen from among pentane-2,4-dione, butan-1-al-3-one, ethyl acetylacetate, methyl acetylacetate, phenylsulphonylacetone, ethyl phenylsulphonylacetate, ethylsulphonylacetone, ethyl cyanoacetate, cyanoacetone, ethyl nitroacetate, nitroacetone, diethyl malonate or hydroxyacetone.

4.Process according to one of Claims 1, 2 or 3, characterised in that the compound having an activated carbon atom is chosen from among methyl acetylacetate or ethyl acetylacetate.